(19) **Europäisches Patentamt European Patent Office Office européen des brevets**

(11) **EP 4 501 459 A1**

(12) **EUROPEAN PATENT APPLICATION**
published in accordance with Art. 153(4) EPC

(43) Date of publication:
05.02.2025 Bulletin 2025/06

(21) Application number: 22935295.0

(22) Date of filing: 30.03.2022

(51) International Patent Classification (IPC):
*B02C 17/18* (2006.01)

(52) Cooperative Patent Classification (CPC):
B02C 25/00; B02C 17/18; B02C 17/1805;
G01L 5/0071; G01N 33/24

(86) International application number:
PCT/JP2022/016122

(87) International publication number:
WO 2023/188150 (05.10.2023 Gazette 2023/40)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: TOSHIBA MITSUBISHI-ELECTRIC
INDUSTRIAL SYSTEMS
CORPORATION
Chuo-ku
Tokyo 104-0031 (JP)

(72) Inventor: IKEDA, Shuzo
Tokyo 104-0031 (JP)

(74) Representative: TBK
Bavariaring 4-6
80336 München (DE)

(54) **DETECTION DEVICE AND DETECTION SYSTEM**

(57) A detection device according to an embodiment detects a presence or absence of a processing material adhered to an interior wall of a drum of a mill pulverizing a mineral. The detection device is configured to store an initial torque detected at a preset initial rotation angle, calculate a maximum torque based on the initial torque, the initial rotation angle, and a rotation angle greater than the initial rotation angle, calculate a ratio of a torque at the rotation angle divided by the maximum torque, compare the ratio and a preset threshold, and determine that the processing material is adhered to the interior wall of the drum when the ratio is equal to the threshold or greater than the threshold.

FIG. 1

## Description

[Technical Field]

An embodiment of the invention relates to a detection device and a detection system that detect the presence or absence of a processing material originating from a mineral an interior wall of a drum of a mill pulverizing a mineral.

[Background Art]

Mineral resources such as ores and the like, after being extracted from mines or the like, are granulated by pulverizers such as ball mills, SAG (Semi-Auto Geneous) mills, AG (Auto Geneous) mills, etc., to have uniform prescribed sizes and shapes. There are cases where the mineral resource fed into the drum of the mill may adhere to the interior wall of the drum in the granulation process. For example, when the operation is interrupted and the drum is stopped for a long period of time while the processing material of the granulation process remains inside the pulverizer, the likelihood of the processing material of the granulation process adhering to the interior wall of the drum is high. In such a case, when starting the rotation of the drum when the operation is restarted, the processing material may detach from the interior wall of the upper portion of the drum due to its own weight, fall to the bottom portion of the drum, and damage the interior wall of the drum.

The operation of the mill is stopped regularly or irregularly, and an inspection or servicing that includes the interior of the drum is performed. When the processing material is adhered to the interior wall of the drum when such an inspection of the drum interior is performed, the adhered processing material is detached from the interior wall of the drum.

The presence or absence of a processing material such as ores or the like adhered to the interior wall of the drum cannot be determined without stopping the operation of the mill and observing the interior of the drum. Frequent stops of the mill operation reduce the productivity of the mineral resource collection and processing processes.

Technology has been developed to detect the presence or absence of a processing material adhered to the interior wall of a drum when operating a mill. For example, a known system measures the drive torque when a processing material is not adhered to the interior wall of the drum, and detects the presence or absence of adhesion of the processing material based on the characteristics of the rotation angle of the electric motor and the drive torque after the mill is restarted (see Patent Literature 1). However, mill drive systems are often designed and optimized for each installation location. Therefore, in a system such as that of Patent Literature 1, the torque characteristics must be acquired each time a mill is installed, and it is difficult to easily detect the presence or absence of the processing material adhered to the interior wall of the drum.

A detection device and a detection system that can easily detect the presence or absence of a processing material adhered to the interior wall of the drum without observing the interior of the drum are desirable.

[Citation List]

[Patent Literature]

[Patent Literature 1]
United States Patent No. 9522400

[Summary of Invention]

[Problem to be Solved by the Invention]

Embodiments of the invention are directed to solve the problems described above, and to provide a detection device and a detection system that can easily detect the presence or absence of a processing material adhered to the interior wall of the drum.

[Means for Solving the Problem]

A detection device according to an embodiment of the invention detects the presence or absence of a processing material adhered to an interior wall of a drum of a mill pulverizing a mineral. The detection device is configured to store an initial torque when the processing material is adhered to the interior wall of the drum, the initial torque being detected at an initial rotation angle of the drum, the initial rotation angle being prescribed, calculate a maximum torque at a rotation angle greater than the initial rotation angle when the processing material is adhered to the interior wall of the drum based on the initial torque, the initial rotation angle, and the rotation angle, calculate a ratio of a torque detected at the rotation angle divided by the maximum torque, compare the ratio and a preset threshold, determine that the processing material is not adhered to the interior wall of the drum when the ratio is less than the threshold, and determine that the processing material is adhered to the interior wall of the drum when the ratio is equal to the threshold or greater than the threshold after the rotation angle of the drum has reached a first rotation angle at which the processing material can detach and fall from the interior wall of the drum, wherein the first rotation angle is greater than the initial rotation angle.

[Effects of the Invention]

According to embodiments of the invention, a detection device and a detection system that can easily detect the presence or absence of a processing material adhered to an interior wall of a drum are realized.

[Brief Description of Drawings]

FIG. 1 is a schematic block diagram illustrating a mill according to an embodiment.
FIG. 2 is a schematic view for describing an operation principle of a detection device according to the embodiment.
FIG. 3 is a schematic view for describing the operation principle of the detection device according to the embodiment.
FIG. 4 is a schematic view for describing the operation principle of the detection device according to the embodiment.
FIG. 5 is an example of a flowchart for describing the operation of the detection device according to the embodiment.
FIG. 6 is a schematic block diagram illustrating the detection device according to the embodiment.

[Modes for Carrying Out the Invention]

Various embodiments are described below with reference to the accompanying drawings.
The drawings are schematic or conceptual; and the relationships between the thickness and width of portions, the proportions of sizes among portions, etc., are not necessarily the same as the actual values. Also, the dimensions and proportions may be illustrated differently among drawings, even when the same portion is illustrated.
In the specification and drawings, components similar to those described previously or illustrated in an antecedent drawing are marked with the same reference numerals; and a detailed description is omitted as appropriate.

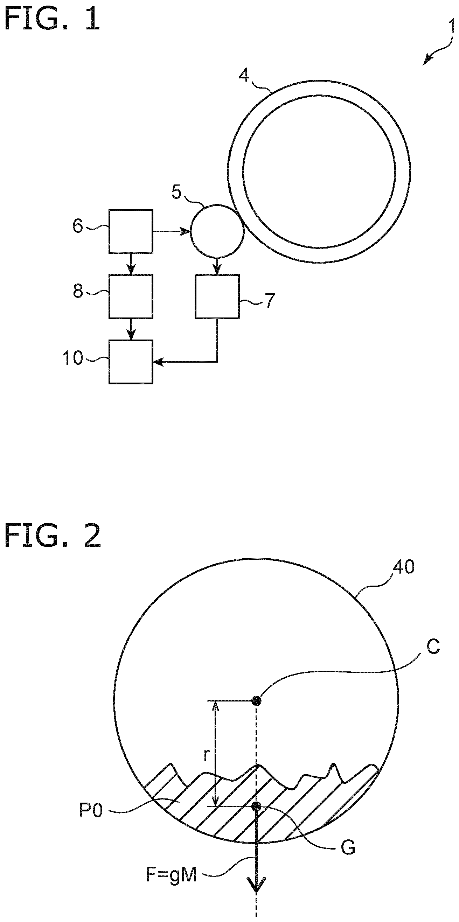

FIG. 1 is a schematic block diagram illustrating a mill according to an embodiment.
As shown in FIG. 1, a detection system 1 includes an electric motor 5, a drive device 6, a rotation angle detector 7, a torque detector 8, and a detection device 10. The detection system 1 is provided together with a mill body 4.
The mill body 4 is connected with the electric motor 5 via a transmission, etc. The mill body 4 includes a rotating drum as described below. The drum is a circular tubular member; and minerals of prescribed sizes are fed into the drum. The drum of the mill body 4 is rotationally driven by the electric motor 5. The electric motor 5 is a DC motor or an AC motor and is, for example, in the case of an AC motor, an induction motor or a synchronous motor. The drive device 6 is connected to the electric motor 5. The drive device 6 applies a prescribed voltage to the electric motor 5, causes a prescribed current to flow in the electric motor 5, and drives the electric motor 5 by the magnetic field generated according to the current.
The drive device 6 drives the electric motor 5 according to a control program and parameters for the control program introduced to a not-illustrated main control device. For example, the drive device 6 drives the electric motor 5 so that the rotational speed follows a speed reference, which is a preset parameter.
The rotation angle detector 7 is connected to the electric motor 5. For example, the rotation angle detector 7 detects the number of rotations per unit time of the electric motor 5, and calculates and outputs the rotation angle of the drum of the mill body 4 based on the reduction ratio of the electric motor 5 and the mill body 4. The rotation angle detector 7 is, for example, a measurement device including a rotary encoder, etc. An appropriate rotary encoder, such as optical, magnetic, or the like is selected. The rotation angle detector 7 may be integrated with the electric motor 5 or may be installed separately from the electric motor 5. The rotation angle detector 7 detects and outputs the number of rotations per unit time of the electric motor 5; and the number of rotations of the electric motor 5 may be converted into the rotation angle of the drum by the detection device 10.
The torque detector 8 is connected to the drive device 6. The torque detector 8 receives input of a signal related to the output current from the drive device 6, and calculates and outputs the value of the torque output by the electric motor 5 based on the output current. The torque detector 8 may be integrated with the drive device 6, or may be installed separately from the drive device 6. Or, the torque detector 8 may include an individual torque detection mechanism independent of the drive device 6.
The detection device 10 that detects the presence or absence of a processing material adhered to the interior wall of the drum is connected to the output of the rotation angle detector 7 and connected to the output of the torque detector 8. The detection device 10 successively receives input of the data of the rotation angle of the electric motor 5 and the data of the torque of the electric motor 5, and detects the presence or absence of the adhesion of the processing material to the interior wall of the drum of the mill body 4.

The detection device 10 calculates the torque when the processing material is adhered to the interior wall of the drum as the maximum value of the torque, and determines that the processing material is not adhered to the interior wall of the drum when the measured torque is sufficiently less than the calculated maximum value of the torque. The detection device 10 determines that there is an adhered material on the interior wall of the drum when the measured torque is near the calculated maximum value of the torque.

The maximum value of the torque when the processing material is adhered to the interior wall of the drum is taken as a maximum torque $Tmax$. The detection device 10 calculates the maximum torque $Tmax$ as follows. Namely, the detection device 10 receives input from the torque detector 8 of an initial torque $TL$ at a preset initial rotation angle $\theta L$. The initial rotation angle $\theta L$ is set to a value that is small enough that the adhesion of the processing material to the interior wall of the drum can be maintained.

The electric motor 5 continues to rotate past the initial rotation angle $\theta L$; and a rotation angle $\theta$ is detected by the rotation angle detector 7 at, for example, constant intervals. The detection device 10 calculates the maximum torque $Tmax$ at the rotation angle $\theta$ by applying the value of the rotation angle $\theta$ and the value of the initial torque $TL$ in Formula (1), described below, each time the rotation angle $\theta$ is detected.

Each time the maximum torque $Tmax$ is calculated, the detection device 10 calculates the ratio with a torque T of the actual measurement. The detection device 10 compares a preset threshold $\alpha$ and $T/Tmax$, and determines that there is no adhered material on the interior wall of the drum when $T/Tmax$ is less than the threshold $\alpha$. When $T/Tmax$ is not less than, the detection device 10 determines that there is an adhered material on the interior wall of the drum.

The operation principle of the detection device 10 will now be described to easily understand the detection device 10 of the embodiment.

FIGS. 2 to 4 are schematic views for describing the operation principle of the detection device according to the embodiment.

FIGS. 2 to 4 show a drum 40 included in the mill body 4 shown in FIG. 1. The drum 40 shown in FIGS. 2 to 4 is illustrated so that the state of the interior is visible. Processing materials P0 to P2 are disposed inside the drum 40. In FIGS. 2 to 4, the drum 40 rotates together with the processing materials P0 to P2 counterclockwise around C. The advancing drawing numbers of FIGS. 2, 3, and 4 indicate that time has elapsed.

In FIGS. 2 to 4, the processing materials P0 to P2 are the same substance with the same mass M. G is the center of gravity when the mass M is taken to be a lumped mass. $r$ is the shortest distance between the center C and the center of gravity G. Although the shapes of the processing materials P0 to P2 may change as the drum 40 rotates, the center of gravity G is taken to be constant at a position separated from the center C by the distance r unless otherwise noted in the following description.

FIG. 2 shows the state of the drum 40 before startup of the mill body 4.

As shown in FIG. 2, the processing material P0 remains at the bottom of the drum 40. The processing material P0 is taken to be adhered to the interior wall of the drum 40. The processing material P0 is subjected to the force of $F = gM$ in the direction of gravity due to its own mass M. The rotation angle of the drum in the state of FIG. 2 is a reference value; and the rotation angle at this time is, for example, 0°. Hereinbelow, the rotation angle is taken to be an angle referenced to 0°.

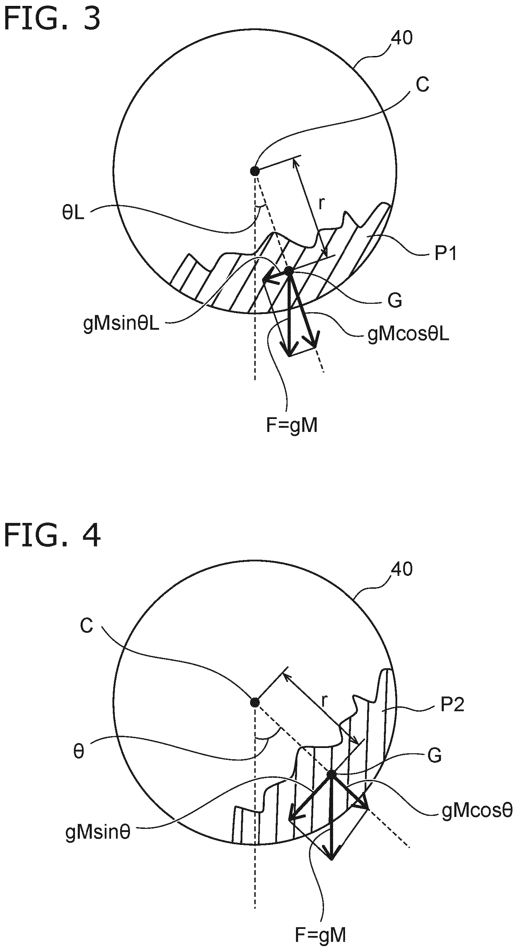

FIG. 3 shows the state in which the mill body 4 has started from the state of FIG. 2, and the drum 40 has rotated the initial rotation angle $\theta L$ counterclockwise.

As shown in FIG. 3, the processing material P1 is adhered to the interior wall of the drum 40; and the initial rotation angle $\theta L$ is the angle between the line segment connecting the center C and the center of gravity G in FIG. 3 and the line segment connecting the center C and the center of gravity G in FIG. 2.

In the state of FIG. 3, the force F due to gravity at the center of gravity G is split into a tangential component and a normal component with respect to the circumference of the drum 40. The tangential component of the circumference of the drum 40 is calculated as $gM\sin\theta L$; and the normal component of the circumference of the drum 40 is calculated as $gM\cos\theta L$.

The processing material P1 generates a moment due to the force $gM\sin\theta L$ in the tangential direction of the circumference of the drum 40. The moment at this time is calculated as $rgM\sin\theta L$ by using the distance r between the center C and the center of gravity G.

In the state of FIG. 3, the drive device 6 generates torque to drive the electric motor 5 to overcome the moment of $rgM\sin\theta L$. The torque at the initial rotation angle $\theta L$ is called the initial torque $TL$. In the detection device 10, the calculations hereinafter are performed by assuming the initial torque $TL$ to be equal to $rgM\sin\theta L$.

FIG. 4 shows the state inside the drum 40 when the drum 40 is rotated further counterclockwise from the initial rotation angle $\theta L$ and has reached the rotation angle $\theta$. The rotation angle $\theta$ is the angle between the line segment connecting the center C and the center of gravity G in FIG. 4 and the line segment connecting the center C and the center of gravity G in FIG. 2.

As shown in FIG. 4, similarly to the case of FIG. 3, the force F due to gravity at the center of gravity G is split into a tangential component and a normal component at the circumference of the drum 40. The tangential component of the circumference of the drum 40 is calculated as $gM\sin\theta$; and the normal component of the circumference of the drum 40 is calculated as $gM\cos\theta$.

In the state of FIG. 4, the processing material P2 generates a moment due to the forced $gM\sin\theta$ in the tangential direction of the circumference of the drum 40. The moment at this time is calculated as $rgM\sin\theta$ by using the distance r between the center C and the center of gravity G.

When the processing material P2 is adhered to the interior wall of the drum 40, the drive device 6 generates torque to drive the electric motor 5 to overcome the moment of $rgM\sin\theta$. When the processing material is not adhered to the interior wall of the drum 40, the drive device 6 can drive the electric motor 5 with sufficiently less torque than $rgM\sin\theta$. Accordingly, $rgM\sin\theta$ can be taken to be the maximum torque $Tmax$ generated by the drive device 6 in the operation of the detection device 10.

The maximum torque $Tmax$ is calculated as follows by applying the initial torque $TL$, the initial rotation angle $\theta L$, and the rotation angle $\theta$ to the following Formula (1).

$$Tmax = rgM\sin\theta$$
$$= rgM\sin\theta L \times (\sin\theta / \sin\theta L)$$
$$= TL \times (\sin\theta / \sin\theta L) \qquad\qquad (1)$$

Here, $\theta > \theta L$.

The initial torque $TL$ is detected by the torque detector 8 and input to the detection device 10. The initial rotation angle $\theta L$ and the rotation angle $\theta$ are detected by the rotation angle detector 7 and input to the detection device 10. The detection device 10 can calculate the maximum torque $Tmax$ using Formula (1).

The threshold $\alpha$ is preset in the detection device 10. The detection device 10 calculates the ratio of the maximum torque $Tmax$ and the measured torque $T$ detected by the torque detector 8, calculates $T/Tmax$ each time the rotation angle $\theta$ is detected, and compares the calculated result and the threshold $\alpha$.

The initial rotation angle $\theta L$ is set to a rotation angle small enough that the state can be maintained in which the processing material is adhered to the interior wall of the drum. At the startup of the electric motor 5, the starting torque needs to be greater than that of steady-state operation. Therefore, the initial rotation angle $\theta L$ is favorably set to a rotation angle after the electric motor 5 has gone past the starting torque, and is set to appropriate values according to the mechanical constants of the electric motor 5 and the mill body 4. For example, the initial rotation angle $\theta L$ is set to about 20°.

The threshold $\alpha$ is set to a more appropriate value by experiment, simulation, etc., according to the mill body 4. $Tmax$ is the maximum value when $\theta = 90°$, and so the threshold $\alpha$ is a value less than 1.

$T/Tmax$ is 1 regardless of the presence or absence of the processing material until the drum 40 rotates past the initial rotation angle $\theta L$ and the processing material starts to deform. Therefore, $T/Tmax$ exceeds the threshold $\alpha$ when the drum 40 exceeds the initial rotation angle $\theta L$, and so the detection device 10 would undesirably determine that the processing material is adhered even when the processing material is not adhered. Therefore, in the detection device 10 of the embodiment, an adhered material presence detection angle (a first rotation angle) $\theta C$ is set at which the presence or absence of the adhered material is detected. Until the rotation angle $\theta$ of the drum 40 reaches $\theta C$, the adhesion of the compared material is not determined even when $T/Tmax$ is equal to the threshold $\alpha$ or greater than the threshold $\alpha$. The adhered material presence detection angle $\theta C$ is selected to be an angle at which the processing material reliably starts to deform when the processing material is not adhered. For example, the adhered material presence detection angle $\theta C$ is set to about 60°.

The series of operations described above will now be described using a flowchart.

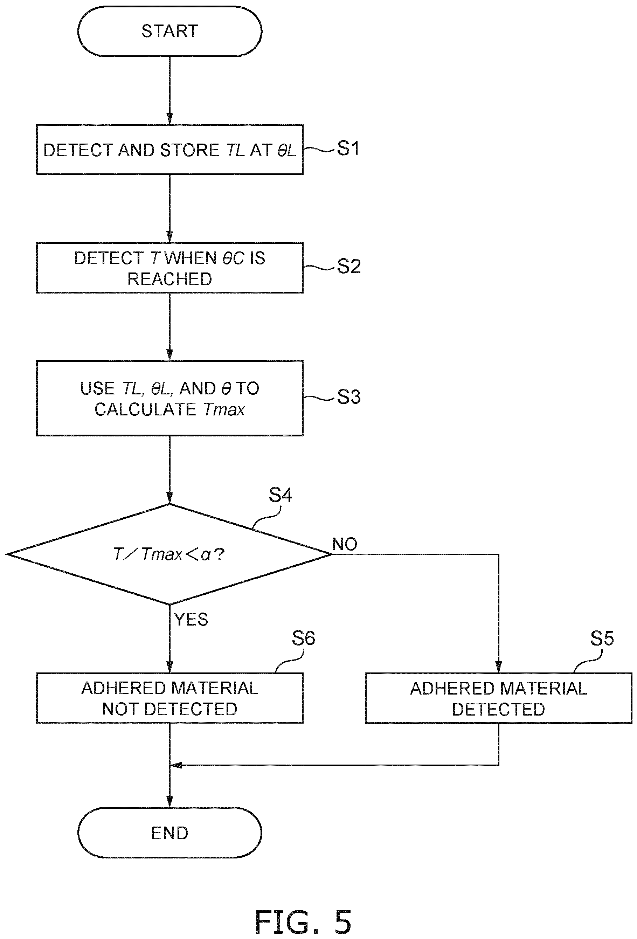

FIG. 5 is an example of a flowchart for describing the operation of the detection device according to the embodiment.

In step S1 as shown in FIG. 5, the detection device 10 receives input of the initial rotation angle $\theta L$ detected by the rotation angle detector 7, receives input of the initial torque $TL$ detected by the torque detector 8, and stores the initial rotation angle $\theta L$ and the initial torque $TL$.

In step S2, the rotation angle detector 7 detects that the rotation angle $\theta$ has reached the adhered material presence detection angle 6C; and the torque detector 8 detects the torque at the rotation angle $\theta$. The detection device 10 receives input of the data of the detected rotation angle $\theta$ and data of the detected torque T.

In step S3, the detection device 10 calculates the maximum torque $Tmax$ using the stored initial rotation angle $\theta L$, the stored initial torque $TL$, and the input rotation angle $\theta$.

In step S4, the detection device 10 calculates $T/Tmax$ and compares the calculation result and the preset threshold $\alpha$. When $T/Tmax$ is less than the threshold $\alpha$, the detection device 10 determines that there is no adhered material in step S6. When the calculated $T/Tmax$ is equal to the threshold $\alpha$ or greater than the threshold $\alpha$, the detection

device 10 determines that an adhered material on the interior wall of the drum 40 is detected in step S5.

The detection device 10 of the embodiment is a computer device that executes the steps of the flowchart shown in FIG. 5; and the computer device stores a program including the steps in a storage device or the like, and when necessary, reads the program. The computer device that realizes the detection device 10 may be a programmable logic controller (PLC). The content of the flowchart shown in FIG. 5 may be applied to the control program executed by the PLC.

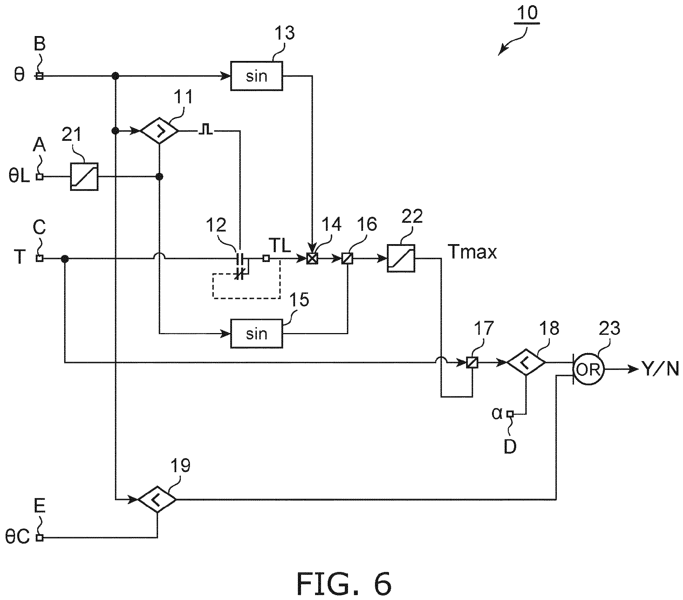

FIG. 6 is a schematic block diagram illustrating the detection device according to the embodiment.

In FIG. 6, the flowchart shown in FIG. 5 is redrawn to illustrate the control program introduced to the PLC.

The operation of the detection device 10 of the embodiment will now be described according to FIG. 6.

As shown in FIG. 6, the detection device 10 includes comparison determination elements 11, 18, and 19, a storage element 12, arithmetic elements 13 to 17, numeric limiting elements 21 and 22, and an OR logic element 23.

The initial rotation angle $\theta L$ is preset in the detection device 10. In the example, the numeric limiting element 21 is located at an input terminal A of the initial rotation angle. The initial rotation angle $\theta L$ that is input to the input terminal A is input to one terminal of the comparison determination element 11.

The numeric limiting element 21 is provided to pre-limit the range of the initial rotation angle $\theta L$, and is set to have, for example, a maximum limit value greater than the rotation angle when the starting torque of the electric motor 5 is generated, and a minimum limit value less than 45°. The setting of the numeric limiting element 21 is arbitrary, and the numeric limiting element 21 may not be provided.

The rotation angle $\theta$ of the drum 40 that is obtained from the electric motor 5 or the rotation angle $\theta$ of the drum 40 that is obtained by another method is detected by the rotation angle detector 7 and input to an input terminal B. The input rotation angle $\theta$ is input to another terminal of the comparison determination element 11.

The comparison determination element 11 outputs a pulse when the magnitude of the rotation angle $\theta$ exceeds the magnitude of the initial rotation angle $\theta L$. The pulse that is output is input to the storage element 12.

The torque T that is detected by the torque detector 8 is input to an input terminal C. The input torque T is input to the storage element 12. The storage element stores the torque T when receiving the pulse output by the comparison determination element 11. Because the pulse output when the rotation angle $\theta$ exceeds the initial rotation angle $\theta L$ is input to the storage element 12, the data that is stored in the storage element 12 is the initial torque $TL$ at the initial rotation angle $\theta L$.

The storage element 12 outputs the initial torque $TL$. The rotation angle $\theta$ that is input to the input terminal B is input to the arithmetic element 13. The arithmetic element 13 performs a sine operation and outputs $\sin\theta$.

The arithmetic element 14 receives input of $\sin\theta$ calculated by the arithmetic element 13 and the initial torque $TL$ from the storage element 12, and outputs the multiplication result $TL\sin\theta$.

The initial rotation angle $\theta L$ that is input from the input terminal A is input to the arithmetic element 15. The arithmetic element 15 performs a sine operation and outputs $\sin\theta L$.

The arithmetic element 16 divides $TL\cdot\sin\theta$ calculated and output by the arithmetic element 14 by $\sin\theta L$ calculated and output by the arithmetic element 15, and outputs the result as the maximum torque $Tmax$. The detailed calculations utilize the derivation process of Formula (1) above. In the example, the numeric limiting element 22 is provided at the output of the arithmetic element 16. The numeric limiting element 22 is provided to exclude inappropriate calculation results due to arithmetic errors generated by the arithmetic elements.

The arithmetic element 17 outputs the torque T detected by the torque detector 8 divided by the maximum torque $Tmax$ calculated and output by the arithmetic element 16.

At one terminal D of the comparison determination element 18, the threshold $\alpha$ is preset and input to the comparison determination element 18. $T/Tmax$ that is calculated and output by the arithmetic element 17 is input to another terminal of the comparison determination element 18. The comparison determination element 18 compares $T/Tmax$ and the threshold $\alpha$, and when $T/Tmax$ is less than the threshold $\alpha$, determines that there is no adhered material on the interior wall of the drum, and outputs a signal (Y) indicating no adhered material. When $T/Tmax$ is equal to the threshold $\alpha$ or greater than the threshold $\alpha$, the comparison determination element 18 determines that an adhered material is on the interior wall of the drum 40, and outputs a signal (N) indicating that there is an adhered material. In the example, the signal (Y) indicating no adhered material corresponds to the logical value "1"; and the signal (N) indicating that there is an adhered material corresponds to the logical value "0".

The output of the comparison determination element 18 is determined based on the magnitude relationship between the rotation angle $\theta$ and the adhered material presence detection angle $\theta C$. The comparison determination between the rotation angle $\theta$ and the adhered material presence detection angle $\theta C$ is performed by the comparison determination element 19. The terminal B is connected to one input of the comparison determination element 19, and inputs the rotation angle $\theta$. The terminal E is connected to another input of the comparison determination element 19, and inputs the preset adhered material presence detection angle $\theta C$.

The output of the comparison determination element 18 is connected to one input of the OR logic element 23; and the output of the comparison determination element 19 is connected to another input of the OR logic element 23.

When the rotation angle $\theta$ is determined to be less than the adhered material presence detection angle $\theta C$, the

comparison determination element 19 outputs the logical value "1". Accordingly, the logical value "1" is input to the OR logic element 23; therefore, the OR logic element 23 outputs the logical value "1", i.e., the signal (Y) indicating no adhered material, regardless of the output of the comparison determination element 18.

When the rotation angle $\theta$ is determined to be not less than the adhered material presence detection angle $\theta C$, the comparison determination element 19 outputs the logical value "0". Accordingly, the OR logic element 23 outputs the logical value of the output of the comparison determination element 18. That is, when the comparison determination element 18 outputs the signal (N) indicating that there is an adhered material, the OR logic element 23 outputs the signal (N) indicating that there is an adhered material. When the comparison determination element 18 outputs the signal (Y) indicating no adhered material, the OR logic element 23 outputs the signal (Y) indicating no adhered material.

Thus, the detection device 10 of the embodiment can determine the presence or absence of the adhesion to the interior wall of the drum of a processing material originating from a mineral.

The detection device 10 described above operates at the startup of the mill body 4 after stopping due to an inspection of the mill body 4, etc. That is, at the startup of the mill body 4 when the drive device 6 starts to drive the electric motor 5, the detection device 10 operates and detects the presence or absence of adhesion of a processing material to the interior wall of the drum. When the adhesion of the processing material is detected, for example, the detection device 10 generates a stop command to stop the drive of the electric motor 5. A not-illustrated main control device that receives the stop command stops the operation of the mill body 4 itself by an interlock. The removal of the adhered material, etc., are safely performed thereby.

The case where the detection device 10 detects the adhesion of the processing material to the interior wall of the drum is not limited to that described above; a command that causes the main control device to perform a processing material removal operation to eliminate the adhesion of the processing material may be generated. For example, the processing material removal operation is an operation in which the main control device causes a rotation to about -90° in the opposite direction of the current rotational direction, and then causes a rotation in the original rotational direction to loosen the processing material and eliminate the adhesion.

When the adhesion of the processing material to the interior wall of the drum is not detected, the detection device 10 may maintain the operating state as-is, or may stop the operation after a preset rotation angle has been passed.

Effects of the detection device 10 of the embodiment will now be described.

In the detection device 10 of the embodiment, the torque when a processing material is adhered to the interior wall of the drum can be calculated as the maximum torque *Tmax* based on the rotation angle detected by the rotation angle detector 7 of the electric motor 5 and the torque detected by the torque detector 8. The maximum torque *Tmax* can be easily calculated using Formula (1). Accordingly, by appropriately setting the threshold $\alpha$, the presence or absence of the adhesion of the processing material to the interior wall of the drum can be easily determined.

By performing the adhesion determination as described above, the detection device 10 of the embodiment does not need to acquire the torque characteristics of the electric motor including the mechanical systems such as the drum and the like for each mill installation condition and type and condition of processing materials; therefore, the installation and adjustment can be easily performed.

Thus, a detection device that can easily detect the presence or absence of a processing material adhered to the interior wall of the drum is realized.

Although several embodiments of the invention are described hereinabove, these embodiments have been presented by way of example only, and are not intended to limit the scope of the inventions. These novel embodiments may be embodied in a variety of other forms; and various omissions, substitutions, and changes may be made without departing from the spirit of the inventions. Such embodiments and their modifications are within the scope and spirit of the inventions, and are within the scope of the inventions described in the claims and their equivalents. Also, the embodiments described above can be implemented in combination with each other.

[Reference Numeral List]

1 mill
4 mill body
5 electric motor
6 drive device
7 rotational speed detector
8 torque detector
10 detection device
11, 18, 19 comparison determination element
12 storage element

13 to 17 arithmetic element
21, 22 numeric limiting element
23 OR logic element
40 drum

**Claims**

1. A detection device detecting a presence or absence of a processing material adhered to an interior wall of a drum at a startup of a mill pulverizing a mineral, the detection device being configured to:

   store an initial torque when a processing material is adhered to the interior wall of the drum, the initial torque being detected at an initial rotation angle of the drum, the initial rotation angle being prescribed;
   calculate a maximum torque at a rotation angle greater than the initial rotation angle when the processing material is adhered to the interior wall of the drum based on the initial torque, the initial rotation angle, and the rotation angle;
   calculate a ratio of a torque detected at the rotation angle divided by the maximum torque;
   compare the ratio and a preset threshold;
   determine that the processing material is not adhered to the interior wall of the drum when the ratio is less than the threshold; and
   determine that the processing material is adhered to the interior wall of the drum when the ratio is equal to the threshold or greater than the threshold after the rotation angle of the drum has reached a first rotation angle at which the processing material can detach and fall from the interior wall of the drum, the first rotation angle being greater than the initial rotation angle.

2. The detection device according to claim 1, wherein

   the calculating of the maximum torque is a calculation according to Formula (1), wherein $Tmax$ is the maximum torque, $TL$ is the initial torque, $\theta$ is the rotation angle, and $\theta L$ is the initial rotation angle.

$$Tmax = TL \times (\sin\theta / \sin\theta L) \qquad (1)$$

3. The detection device according to claim 1, further comprising:
   generating a command to stop an operation of the mill when it is determined that the processing material is adhered to the interior wall of the drum.

4. The detection device according to claim 1, further comprising:
   generating a command to cause an operation of the mill to be the processing material removal operation when it is determined that the processing material is adhered to the interior wall of the drum.

5. A detection system, comprising:

   the detection device according to claim 1;
   an electric motor driving the drum of the mill;
   a drive device driving the electric motor;
   a rotation angle detector detecting a rotation angle of the electric motor; and
   a torque detector detecting a torque output by the drive device,
   the mill being one of a ball mill, a SAG mill, or an AG mill,
   the rotation angle detector successively detecting the rotation angle and transmitting the rotation angle to the detection device,
   the torque detector detecting the initial torque and transmitting the initial torque to the detection device, and successively detecting the torque at the rotation angle and transmitting the torque to the detection device,
   the detection device calculating the ratio, comparing the ratio and the threshold, and determining a magnitude relationship between the ratio and the threshold each time the detection device receives the torque at the rotation angle.

# FIG. 1

# FIG. 2

FIG. 3

FIG. 4

START

DETECT AND STORE $TL$ AT $\theta L$ —— S1

DETECT $T$ WHEN $\theta C$ IS REACHED —— S2

USE $TL$, $\theta L$, AND $\theta$ TO CALCULATE $Tmax$ —— S3

S4

$T / Tmax < \alpha$ ?

NO

YES

S6

ADHERED MATERIAL NOT DETECTED

S5

ADHERED MATERIAL DETECTED

END

FIG. 5

FIG. 6

## INTERNATIONAL SEARCH REPORT

| | International application No. |
|---|---|
| | **PCT/JP2022/016122** |

**A. CLASSIFICATION OF SUBJECT MATTER**

*B02C 17/18*(2006.01)i
FI: B02C17/18 Z

According to International Patent Classification (IPC) or to both national classification and IPC

**B. FIELDS SEARCHED**

Minimum documentation searched (classification system followed by classification symbols)

B02C1/00-B02C25/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2018/010880 A1 (SIEMENS AKTIENGESELLSCHAFT,DE) 18 January 2018 (2018-01-18) in particular, p. 7, line 12 to p. 10, line 6, fig. 1, 2 | 1-5 |
| A | US 2003/0052205 A1 (TIRSCHLER, Ehrenfried Albert) 20 March 2003 (2003-03-20) in particular, paragraphs [0106]-[0134], fig. 1-9 | 1-5 |
| A | JP 62-37086 A (SIEMENS AKTIENGESELLSCHAFT,DE) 18 February 1987 (1987-02-18) | 1-5 |
| A | US 2007/0145168 A1 (METSO MINERALS INDUSTRIES, INC.) 28 June 2007 (2007-06-28) | 1-5 |
| A | JP 2017-513694 A (SIEMENS AKTIENGESELLSCHAFT,DE) 01 June 2017 (2017-06-01) | 1-5 |
| E, A | JP 2022-83314 A (NAGAO SYSTEM KK) 03 June 2022 (2022-06-03) | 1-5 |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **10 June 2022** | **21 June 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**EP 4 501 459 A1**

<table>
<tr><th colspan="2">INTERNATIONAL SEARCH REPORT<br>Information on patent family members</th><th colspan="2">International application No.<br><br>**PCT/JP2022/016122**</th></tr>
<tr><th>Patent document<br>cited in search report</th><th>Publication date<br>(day/month/year)</th><th>Patent family member(s)</th><th>Publication date<br>(day/month/year)</th></tr>
<tr><td>WO 2018/010880 A1</td><td>18 January 2018</td><td>EP 3269453 A1<br>CA 3030596 A<br>in particular, p. 8, line 8 to p.<br>11, line 11, fig. 1, 2<br>CN 109562388 A</td><td></td></tr>
<tr><td>US 2003/0052205 A1</td><td>20 March 2003</td><td>US 2006/0113416 A1<br>CA 2402125 A1</td><td></td></tr>
<tr><td>JP 62-37086 A</td><td>18 February 1987</td><td>DE 3528409 A1<br>CH 670016 A5</td><td></td></tr>
<tr><td>US 2007/0145168 A1</td><td>28 June 2007</td><td>US 2011/0297768 A1</td><td></td></tr>
<tr><td>JP 2017-513694 A</td><td>01 June 2017</td><td>US 2018/0169663 A1<br>WO 2015/144444 A1<br>EP 2923767 A1<br>CA 2943579 A1<br>KR 10-2016-0134809 A<br>CN 106102919 A</td><td></td></tr>
<tr><td>JP 2022-83314 A</td><td>03 June 2022</td><td>(Family: none)</td><td></td></tr>
</table>

Form PCT/ISA/210 (patent family annex) (January 2015)

14

**EP 4 501 459 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9522400 B